# EUROPEAN PATENT APPLICATION

(11) **EP 2 468 270 A1**
(43) Date of publication of application: **27.06.2012**
(21) Application number: 10196271.0
(22) Date of filing: 21.12.2010
(51) Int. Cl.: A61K 31/192, A61P 17/04

(54) **(R)-2-(3-fluoro-4-phenylphenyl)propionic acid for use in the treatment of skin diseases**

(71) Applicant: GALENpharma GmbH, 24109 Kiel (DE)
(72) Inventor: Mehnert, Jörg, 24109 Kiel (DE); Soblik, Hanns, 24109 Kiel (DE); Piotrowiak, Ralf, 24109 Kiel (DE)
(74) Representative: UEXKÜLL & STOLBERG

(57) **Abstract**

The present invention relates to (R)-2-(3-fluoro-4-phenylphenyl)propionic acid (tarenflurbil) or a pharmaceutically acceptable salt, solvate or ester thereof for use in the treatment of inflammation and/or itching of the skin as well as a pharmaceutical composition comprising tarenflurbil.

## Description

The present invention relates to (R)-2-(3-fluoro-4-phenylphenyl)propionic acid (tarenflurbil) for use in the treatment of skin diseases and pharmaceutical compositions comprising tarenflurbil.

Skin diseases and, in particular, inflammatory skin diseases today represent a widespread and unsolved problem. The most frequent non-infectious, inflammatory skin diseases include atopic dermatitis and psoriasis.

In Germany alone, between 3.5 and 5 million people suffer from atopic dermatitis (syn. neurodermatitis, asthmaeczema, atopic eczema, chronic constitutional eczema, endogenous eczema, prurigo Besnier, neurodermatitis diffusa, intrinsic eczema, infantile eczema, allergic eczema). Frequency has increased significantly during the last few decades. Atopic dermatitis is one of the most frequent childhood diseases (10-20%) (Dermatologie und Venerologie, Ed. Braun-Falco et al., 5th edition, 2005, p. 381). The incidence of psoriasis (syn. psora, arthritis psoriatica, psoriatic osteoarthropathy, arthropathia psoriatica, rhumatisme psoriasique, chronic arthritis psoriatica, psoriaris vulgaris, psoriasis nummularis, generalized psoriasis pustulosa, impetigo herpetiformis, psoriasis pustulosa of the Zumbusch type, acrodermatitis continua suppurativa (acrodermatitis continua suppurativa of Hallopeau), psoriasis pustulosa palmoplantaris, guttate psoriasis, psoriatic arthropathy, psoriasis inversa) is reported to be approximately 2% in Europe and the USA (Dermatologie und Venerologie, Ed. Braun-Falco et al., 5th edition, 2005, p. 476).

Chronic inflammatory skin diseases such as psoriasis or atopic dermatitis represent a severe physical and emotional burden on those affected. Thus far it has not been possible to cure such skin diseases, which means that therapy has been restricted to alleviating the symptoms. Today there are no medicinal products or non-medicamentous measures for treating atopic dermatitis which make possible a long-term cure of the disease (Dermatologie und Venerologie, Ed. Braun-Falco et al., 5th edition, 2005, p. 391).

The pharmacotherapy of chronic, inflammatory skin diseases can be carried out systemically (e.g. by oral administration or injection of a medicinal product) or topically (i.e. applied externally to the skin in targeted manner), wherein the topical therapy is preferred because of the lower risk of side effects. With such a targeted, topical therapy, the active ingredient is transported to its site of action in the tissue affected preferably without noticeably burdening other tissues or organs that are not involved.

The commonly used topical standard therapy often reaches its limits in patients suffering from severe forms of psoriasis or atopic dermatitis and side effects can occur during long-term treatment.

Today the following topical therapeutic agents are commonly used for the treatment of inflammatory skin diseases.

Corticosteroid-containing ointments are used for acute inflammatory skin diseases because of their strongly anti-inflammatory effect. However, cortisol is at the same time a hormone occurring naturally in the body which performs many regulatory functions in the metabolism and in the immune system. Thus, during more prolonged topical treatments, undesired side effects can occur on the skin, such as e.g. teleangiectasias, striae, steroid acne, changes in skin pigmentation, bacterial infections and hypertrichosis. Furthermore, corticoids can lead to epidermal atrophy and thinning of the epidermis making it easy for the latter to be damaged. Systemic side effects in the case of particularly high doses of strong and very strong corticoids include *inter alia* water retention in the tissue (edema) and thus weight increase, weakening of the immune defence, promotion of the development of stomach ulcers, promotion of the development of and exacerbation of existing diabetes mellitus, promotion of the development and exacerbation of an existing bone atrophy (osteoporosis). Following the abrupt discontinuation of a local glucocorticoid therapy, a rebound effect may occur, in which the skin symptoms in question return with greater severity than before the treatment and then not infrequently also respond more poorly than previously to glucocorticoids. Children in particular are more susceptible than are adult patients to a glucocorticoid-induced suppressive effect on the hypothalamus-hypophyseal-adrenal axis and to Cushing's syndrome, because the ratio of skin surface to body weight is greater in children. This extensive list of side effects, some of which are also very serious, has given rise to a "cortisone phobia" in most patients and in some members of the medical profession.

Immune modulators and calcineurin inhibitors, e.g. tacrolimus and pimecrolimus, are two anti-inflammatory medicaments whose therapeutic efficacy can be compared with that of the likewise anti-inflammatory cortisone topical agents, but which do not cause skin atrophy. The active ingredient tacrolimus is known from transplantation medicine, where it is used to inhibit the immune system. However, this group of medicaments has proved to have the major disadvantage that it is clearly less effective compared with the corticoids which are usually applied topically. Furthermore, there has been discussion of carcinogenicity in connection with exposure to light. This group of medicaments has thus become established more as a remedy of second choice, particularly as the high price of the products limits application over a large area.

Systemic administration of anti-inflammatory medicinal products is considered only in rare cases when symptoms are particularly severe, e.g. glucocorticoids as an initial short-term treatment, furthermore immunosuppressants such as cyclosporin A, azathioprine or mycophenolate mofetil. The side effects are numerous and affect the most diverse organs such as e.g. kidneys, liver and gastrointestinal tract; skin, musculature, haematopoietic system, immune system, cardiovascular system, nervous system and sensory organs.

More recent observations indicate that the systemic administration of biologics such as e.g. omalizumab and infliximab can also lead to an improvement in symptoms. However, these are to be used with care, as serious side effects can occur and they therefore require further research (Bieber, Th., Atopic Dermatitis, Ann. Dermatol. Vol. 22, No. 2, 2010, pp. 125-135).

When administered systemically, antihistamines alleviate itching in skin diseases. They block the action of the histamine released from the mast cells which is involved in causing itching. However, the antipruriginous action of these substances is highly controversial in the case of topical administration having regard to eczematous itching. (Dermatologie und Venerologie, Ed. Braun-Falco et al., 5th edition, 2005, p. 393).

Antibiotics are used if bacterial infections of the skin occur, especially following scratch injuries. Such a bacterial infection is rapidly controlled by short-term treatment with antibiotics. However, this successfully controls only the effects accompanying a skin disease, and not the skin disease as such.

Consequently, it is an object of the present invention to provide a new treatment of skin diseases and in particular inflammatory skin diseases which treatment is efficient, but has few side-effects and is thus safe.

Surprisingly, this object is solved by the use of tarenflurbil for the manufacture of a medicament for the treatment of skin diseases. The present invention also relates to a pharmaceutical formulation of tarenflurbil.

Tarenflurbil, i.e. (*R*)-2-(3-fluoro-4-phenylphenyl)propionic acid (syn. (*R*)-2-flurbiprofen, R-flurbiprofen, flurizan, (*R*)-(-)-2-fluoro-α-methyl-4-biphenylacetic acid, (*R*)-2-fluoro-α-methyl-(1,1'-biphenyl)-4-acetic acid, (*R*)-2-(2-fluoro-4-biphenylyl)propionic acid), is the R-enantiomer of flurbiprofen which is a member of the phenylalkanoic acid derivative family of non-steroidal anti-inflammatory drugs (NSAIDs).

Flurbiprofen racemate is used as NSAID for diseases of the soft tissue and musculoskeletal system, e.g. ankylosing spondylitis, osteoarthritis, rheumatoid arthritis, pulled muscles and strains, post-operative pain, dysmenorrhoea and migraine. Flurbiprofen racemate is furthermore used for sore throat and inflammation of the throat area, as well as in eye drops (Sweetman S (Ed.), Martindale: The complete drug reference, London, Pharmaceutical Press, electronic version, October 6, 2010).

Flurbiprofen also is and has been used as a racemate for treating pain and inflammation (in Germany: e.g. Dobendan^{®} Direkt (sore-throat lozenges), OCUFLOR O.K.^{®} (eye drops for inflammatory eye diseases); in the USA: ANSAID^{®} (arthritis tablets); in the UK and Switzerland: FROBEN^{®} (dragées for treating pain, inflammation and fever); in Italy, South Africa and Portugal: TRANSACT^{®} LAT (transdermal plasters with flurbiprofen racemate for the systemic treatment of pain of the musculoskeletal system)) (Sweetman S (Ed.), Martindale: The complete drug reference, London, Pharmaceutical Press. electronic version, October 6, 2010). However, S-flurbiprofen was always considered to be the actual active component whilst the 50% R-flurbiprofen also contained was regarded as a harmless impurity from the synthesis and isomeric ballast.

Geisslinger and colleagues at the University of Frankfurt were the first to demonstrate that R-flurbiprofen does indeed have a pharmacological action (EP 0 607 128; Geisslinger et al., R-flurbiprofen: isomeric ballast or active entity of the racemic compound?, Agents Actions Suppl. 1993, 44, 31-36). Geisslinger and colleagues discovered that, following systemic administration in a higher dose than when administered in the racemate, R-flurbiprofen exhibits a pain- and inflammation-inhibiting action which is not based on cyclooxygenase inhibition, as is the case with S-flurbiprofen. This finding was confirmed by Wechter in WO 00/13684, who assumed an inhibition of the COX-2-mRNA synthesis by R-flurbiprofen.

R-flurbiprofen was also shown to be effective with colorectal cancer in animal experiments (WO 96/28148). At present a Phase IIa trial is underway to examine whether topical tarenflurbil can be safely applied in the case of patients with non-melanoma skin cancer (Australian New Zealand Clinical Trials Registry, ACTRN1206000192583, Date registered 24/05/2006, Title: A Phase IIa safety study of topical R-flurbiprofen in patients with a history of non-melanoma skin cancer). WO 03/061713 describes the chemopreventive effect of arylpropionic acids including flurbiprofen in the prevention of UV-induced skin cancer and pre-cancerous lesions. This preventive effect is assumed to be supported by various studies and experiments comprising the exposure of hairless mice to a combination of UV-A and UV-B light. However, Leipold et al. (Chirality 2004, 16(6), 397-387) has subsequently shown a considerable bioinversion of R-flurbiprofen into S-flurbiprofen in mice so that no particular effect of R-flurbiprofen can be derived from the WO 03/061713 experiments.

The finding that, in animal experiments (rats) with R-flurbiprofen the activation, due to inflammation, of the transcription factor NFkappaB in the nervous system is inhibited, leading to the reduction of pain and inflammation in the neuronal system, was first reported in EP 1 154 766.

Great hopes were previously also placed in R-flurbiprofen for treating neurological diseases, such as Alzheimer's disease. However, this substance proved ineffective against this disease in extensive clinical Phase III studies (WO 2005/065069; Imbimbo BP, Why did tarenflurbil fail in Alzheimer's disease?, J Alzheimer Dis. 2009, 17(4), 757-760).

EP 1 322 305 describes that tarenflurbil influences AP-1 binding to DNA. Moreover, it is reported that tarenflurbil reduces cyclin-B-1 expression, inhibits the G-1 phase of tumour cells and thus influences the cell cycle and apoptosis (Grösch S. et al., Induction of apoptosis by R-flurbiprofen in human colon carcinoma cells: involvement of p53, Biochemical Pharmacology 2005, 69(5), 831-839).

Recently it was further found that R-flurbiprofen is effective not only for nociceptive pain, but also for neuropathic pain (WO 2009/083115). It was also recently recognized that tarenflurbil reduces neuropathic pain via the modulation of the endogenous cannabinoid system (Bishay P. et al., R-flurbiprofen reduces neuropathic pain in rodents by restoring endogenous cannabinoids, PLoS One, 2010 May 13, 5(5) , e10628).

Tarenflurbil did prove effective in the case of bradykinin-induced nociceptor sensitization to heat in isolated rat skin (Pethö G. et al., Bradykinin-induced nociceptor sensitization to heat is mediated by cyclooxygenase products in isolated rat skin, Eur. J Neurosci. 2001, 14(2), 210-218). However, the authors attributed the effect to the S-flurbiprofen also contained as impurity.

WO 92/05768 discloses the use of S-flurbiprofen for the prevention of UV erythema. Administration of S-flurbiprofen is described to be more effective compared to the same dose of flurbiprofen in its racemic form reflecting that the S-enantiomer is the active form of flurbiprofen.

Using the CGRP stimulation model on isolated rat skin, Averbeck et al. showed that S-flurbiprofen is effective and R-flurbiprofen is ineffective to inhibit the release of CGRP (Calcitonin Gene-Related Peptide) which causes neurogenic inflammation and, in this connection, vasodilation (Averbeck B et al., Inflammatory mediators do not stimulate CGRP release if prostaglandin synthesis is blocked by S(+)-flurbiprofen in isolated rat skin, In-flamm. Res. 2003, 52(12), 519-523).

In summary, the pharmacological effects previously reported in connection with administration of flurbiprofen racemate to the skin are attributed to the known cyclooxygenase inhibitory activity of S-flurbiprofen, while R-flurbiprofen is regarded as inactive isomeric ballast. Moreover, following systemic administration R-flurbiprofen is reported to be useful in the treatment of cancer, Alzheimer's disease, nociceptive and neuropathic pain and rheumatism.

Thus, it has surprisingly been found that tarenflurbil can suitably be used for the manufacture of a medicament for the treatment of skin diseases and in particular inflammatory skin diseases.

Accordingly, in a first aspect the present invention relates to tarenflurbil or a pharmaceutically acceptable salt, solvate or ester thereof for use in the treatment of a skin disease.

Preferably, the skin disease to be treated is associated with inflammation and/or itching. Furthermore, it is preferred that the treatment comprises treatment of the inflammatory and/or itching component of a skin disease. Thus, the invention particularly relates to tarenflurbil or a pharmaceutically acceptable salt, solvate or ester thereof for use in the treatment of inflammation and/or itching of the skin.

It is further preferred that the skin disease to be treated is a non-hyperproliferative skin disease, more preferably a skin disease selected from the group consisting of non-hyperproliferative inflammatory skin disorders, non-hyperproliferative skin intolerance reactions and skin appendages diseases. Thus, the invention also relates to tarenflurbil or a pharmaceutically acceptable salt, solvate or ester thereof for use in the treatment of a non-hyperproliferative skin disease.

As used herein, the term "non-hyperproliferative" refers to a disease, wherein the skin exhibits normal cell proliferation. In particular, the DNA synthesis and mitotic activity in the basal section of the epidermis does not show any substantially increased activity leading to a pathological condition, such as more than the threefold activity, compared to healthy skin. Thus, the skin disease to be treated according to this embodiment is particularly not associated with abnormal cell growth and does particularly not include psoriasis, hyperkeratosis, actinic keratosis and seborrheic dermatitis.

Furthermore, as used herein, the terms "inflammatory skin disorders", "skin intolerance reactions" and "skin appendages diseases" particularly refers to the corresponding terms used in Dermatologie und Venerologie, Ed. Braun-Falco et al., 5th edition, 2005, Section III, Chapters 23-28; Section IV, Chapters 32-37; Section V, Chapter 38 and Section IX, Chapter 61.

Moreover, it is preferred that the treatment according to the first aspect of the invention is effected by inhibiting at least one inflammation mediator selected from the group consisting of prostaglandins, leukotrienes, BDNF (brain-derived neurotrophic factor) and TGFβ (transforming growth factor β). It has surprisingly been found that tarenflurbil or a pharmaceutically acceptable salt, solvate or ester thereof is able to reduce the quantity and/or concentration of these inflammation mediators in the course of treatment and thus has a favourable therapeutic effect on the usual course of diseases associated with these mediators of inflammation. It is particularly preferred that the treatment is effected by inhibiting at least one inflammation mediator selected from the group consisting of prostaglandin E2 and cysteinyl leukotrienes like LTC4, LTD4 and LTE4.

Preferably, the skin disease to be treated is a non-infectious skin disease, i.e. a disease which does not etiologically result from the presence of parasite species, such as e.g. pathogenic viruses, prions, microbial agents like bacteria and fungi, and/or other parasite species.

In another preferred embodiment, the skin disease to be treated is associated with IgE (Immunoglobulin E) elevation and/or increased Th2 cytokine expression. In particular, the disease is associated with increased levels of Th2 cytokines such as interleukin-4 (IL-4) which in turn result in increased production of IgE.

Typically, the skin disease to be treated can be an acute or chronic skin disease which is commonly treatable with topical corticosteroids.

Preferably, the skin disease to be treated in accordance with the first aspect of the invention is selected from the group consisting of erythematous diseases, papular and lichenoid diseases (including lichen rubber like lichen rubber planus, lichenoid exanthema, graft-versus-host disease of the skin), pustular diseases, psoriasis, UV erythema, atopic dermatitis, urticaria (including acute urticaria, chronic urticaria, autoimmune urticaria, physical urticaria like pressure urticaria, heat urticaria, cold urticaria and light urticaria, contact urticaria, adrenergic urticaria and aquagenic urticaria), angioedema, contact dermatitis (including irritant dermatitis like acute toxic contact eczema and chronic toxic contact eczema, and allergic contact dermatitis like acute allergic contact dermatitis and chronic allergic contact eczema), dyshidrosis, pruritus, prurigo, acne and rosacea. For example, the skin disease can be selected from the group consisting of non-hyperproliferative erythematous diseases, papular and lichenoid diseases (including lichen rubber like lichen rubber planus, lichenoid exanthema, graft-versus-host disease of the skin), pustular diseases, UV erythema, atopic dermatitis, urticaria (including acute urticaria, chronic urticaria, autoimmune urticaria, physical urticaria like pressure urticaria, heat urticaria, cold urticaria and light urticaria, contact urticaria, adrenergic urticaria and aquagenic urticaria), angioedema, contact dermatitis (including irritant dermatitis like acute toxic contact eczema and chronic toxic contact eczema, and allergic contact dermatitis like acute allergic contact dermatitis and chronic allergic contact eczema), dyshidrosis, pruritus, prurigo, acne and rosacea.

More preferably, the skin disease to be treated is selected from the group consisting of atopic dermatitis, psoriasis, UV erythema, pruritus, allergic skin diseases, such as allergic contact dermatitis and allergic urticaria, and acne. For example, the skin disease to be treated can be selected from the group consisting of atopic dermatitis, UV erythema, pruritus, allergic skin diseases, such as allergic contact dermatitis and allergic urticaria, and acne. With respect to the treatment of acne, it is particularly preferred to use tarenflurbil in the supportive treatment of acne to normalize sebum production.

Most preferably, the skin disease to be treated in accordance with the first aspect of the invention is atopic dermatitis.

It has unexpectedly been found in clinical therapy trials that tarenflurbil is very effective in the treatment of atopic dermatitis. Preferably, the effectiveness of tarenflurbil in treatment of atopic dermatitis is comparable with that of corticosteroids.

Tarenflurbil has been shown not to be converted to S-flurbiprofen in humans (Geisslinger et al., New insights into the site and mode of antinociceptive action of flurbiprofen enantiomers, J Clin Pharmacol. 1996, 36(6), 513-520). Thus, the pharmacological effects achieved with tarenflurbil in accordance with the present invention are not to be explained by secondary effects of S-flurbiprofen formed from tarenflurbil. In addition, side effects otherwise resulting from S-flurbiprofen such as occult bleeding in the gastrointestinal tract, stomach ache, nausea and vomiting, diarrhoea, irritations of the oral mucosa, palsy (paraesthesia), headache, fatigue, vertigo, visual disorders, hearing disturbances, xerostomia, water retention in the tissue (edema), constipation, hypersensitivity reactions of the skin such as erythema, itching (pruritus), rashes (urticaria or purpura) and ringing in the ears (tinnitus) are avoided. In contrast to this, tarenflurbil shows a satisfactory tolerability and no side-effects.

It has been proven particularly preferred that the tarenflurbil or a pharmaceutically acceptable salt, solvate or ester thereof is in a form for topical administration.

Suitable formulations of tarenflurbil, and in particular suitable topical formulations of tarenflurbil, are described below in connection with the second aspect of the invention.

Accordingly, the tarenflurbil or a pharmaceutically acceptable salt, solvate or ester thereof can be in a form of a topical pharmaceutical composition selected from the group consisting of liquid or semi-solid formulations (including a gel, solution, cream, foam, lotion, paste, powder, spray solution or ointment) and plasters.

It is preferred that the topical pharmaceutical composition comprises tarenflurbil or a pharmaceutically acceptable salt, solvate or ester thereof and at least one penetration enhancer. Preferably, the topical pharmaceutical composition is in a form of a solution. It is also preferred that the topical pharmaceutical composition comprises tarenflurbil or a pharmaceutically acceptable salt, solvate or ester thereof, a solvent, a penetration enhancer and optionally further excipients. In a particularly preferred embodiment, the topical pharmaceutical composition comprises tarenflurbil or a pharmaceutically acceptable salt, solvate or ester thereof, propylene glycol, isopropyl alcohol, lecithin, optionally Macrogol 7 glycerol cocoate and optionally further excipients.

It has been found that tarenflurbil is particularly suitable for topical treatment of skin diseases. The studies including clinical therapy trials carried out by the present inventors show that tarenflurbil is not only very effective for skin diseases, but in addition does not produce the sometimes very strong side effects which typically occur in pharmacotherapies of such diseases. Thus, tarenflurbil allows a very effective and safe therapy of skin diseases with few or even no side effects which is very advantageous in particular when treating children.

Besides tarenflurbil for use in the treatment of a skin disease by topical administration, another embodiment of the invention is directed to tarenflurbil or a pharmaceutically acceptable salt, solvate or ester thereof for use in the treatment of skin diseases, wherein the tarenflurbil is in a form for systemic administration. The systemic administration of tarenflurbil can be effected by means of tablets, capsules, suppositories, injections, infusions or other suitable administration forms and can therefore also be a very suitable therapy. In particular, a systemic administration of tarenflurbil can be useful if rather large skin areas of the body are affected by the skin disease to be treated.

Typically, the tarenflurbil or the salt, solvate or ester thereof can be present in a pharmaceutical composition in an amount of 0.001 wt.-% to 80 wt.-%, preferably 0.1 wt.-% to 20 wt.-% and more preferably 0.5 wt.-% to 4 wt.-%, based on the total weight of the composition. Further preferred characteristics of tarenflurbil and/or the pharmaceutical composition to be used in the first aspect of the invention are described below in connection with the second aspect of the invention.

Moreover, the tarenflurbil or the salt, solvate or ester thereof can be administered once to several times daily. Preferably, the tarenflurbil or the salt, solvate or ester thereof is administered once daily. The treatment can range from a short-term treatment like a single administration of tarenflurbil or administration of tarenflurbil over a period of few days to a long-term therapy over many years.

In a second aspect, the present invention relates to a pharmaceutical composition comprising tarenflurbil or a pharmaceutically acceptable salt, solvate or ester thereof.

The pharmaceutical composition can be in the form of tablets, capsules, suppositories, injections, infusions or other suitable administration forms. In a preferred embodiment, the pharmaceutical composition according to the invention is in the form of a topical formulation, i.e. a formulation suitable for topical application of tarenflurbil. Examples of suitable topical formulations include, but are not limited to liquid and/or semi-solid formulations like gels, solutions such as spray solutions, creams, foams, lotions, paste, powders, ointments etc. and tarenflurbil-containing plasters.

Preferably, the pharmaceutical composition is in the form of a solution such as a spray solution. As used herein the term "spray solution" refers to a solution which can easily be handled with a spray or foam system to administer the composition to large (e.g. back, crook of the arm) as well as small skin areas (e.g. face, scalp). Thus, the solution has preferably a viscosity which allows for atomization of the solution using an ordinary mechanical pump spray apparatus. In particular, the viscosity of the solution ranges from 1 mPa·s to 100 mPa·s, preferably from 2 mPa·s to 50 mPa·s, more preferably from 2 mPa·s to 40 mPa·s, when measured using a cone/plate viscometer according to Ph.Eur. 2.2.10.

It is particularly preferred that the pharmaceutical composition according to the invention comprises
(a) tarenflurbil or a pharmaceutically acceptable salt, solvate or ester thereof,
(b) at least one solvent,
(c) at least one penetration enhancer, and
(d) optionally further excipients.

The component (a) of the composition is a drug selected from tarenflurbil or a pharmaceutically acceptable salt, solvate or ester thereof. Examples of useful salts are the addition salts with inorganic or organic bases. Exemplary basic salts include ammonium salts, alkali metal salts such as sodium, lithium and potassium salts, alkaline earth metal salts such as calcium and magnesium salts, salts with organic bases like organic amines such as dicyclohexylamines, t-butyl amines, and salts with amino acids such as arginine and lysine and the like. Examples of suitable solvents for preparing solvates of tarenflurbil include water and ethanol. Preferably, the drug (a) is tarenflurbil.

The enantiomeric purity of the drug (a) is preferably not less than 10% e.e. and more preferably not less than 30% e.e. In particularly preferred embodiments, drug (a) having a high enantiomeric purity of not less than 50% e.e., preferably not less than 80% e.e., more preferably not less than 95% e.e. like 97.5% e.e. and most preferably not less than 99% e.e. is used.

It is preferred that the composition comprises, based on the total weight of the composition, 0.05 to 15 wt.-%, more preferably 0.1 to 5 wt.-% and most preferably 1 to 4 wt.-% of drug (a).

It is also preferred that a single application dose of the pharmaceutical composition according to the invention, such as 1 to 3 spray strokes applying the composition in form of a solution, comprises 0.1 to 200 mg, preferably 0.5 to 100 mg and most preferably 1 to 50 mg like 1 to 10 mg of drug (a).

In addition to tarenflurbil, the composition according to the invention can comprise a second active ingredient selected from the group consisting of dermatic agents, corticoids, antiallergic agents, local anesthetics, chemotherapeutic agents, quinine, fungicides, thalidomide, serotonin, eicosanoids, analgesics, anticonvulsants, non-steroidal anti-inflammatory drugs including S-flurbiprofen, leukotrienes, leukotriene inhibitors, androgens, androgen antagonists, opioid receptor antagonists, anticoagulants, platelet aggregation inhibitors, histamine blockers, regulatory and enzymatic peptides and proteins, nucleic acids (single strand and double strand DNA, single strand and double strand RNA, snRNA, DNA oligo-nucleotides, RNA oligo-nucleotides), oligo-peptides, anti-pruritics, prostaglandins, prostaglandine inhibitors, antiviral drugs, antimicrobials, prion antagonists, immunosuppressive agents, hormones, agents for the treatment of warts and wounds, vitamins, agents for the treatment of circulatory disorders, agents for the treatment of neuropathy, plant extracts, analeptics, general anesthetics, muscle relaxants, antiemetics, antiparasitics, calcium antagonists, diuretics, disinfectants, micronutrients, antiinfectives, cytostatic drugs, antimetabolites, hormone antagonists and immunomodulators as well as salts, solvates and derivatives thereof.

The component (b) of the composition according to the invention is at least one solvent. Preferably, the solvent (b) is capable of dissolving tarenflurbil and providing a solution having a suitable viscosity for spray applications. In a preferred embodiment, the solvent (b) is selected from the group consisting of water and alcohols having 1 to 3 hydroxyl groups and mixtures thereof. In a further preferred embodiment, the solvent (b) is selected from the group consisting of water, glycols, lower alcohols and mixtures thereof. In a particularly preferred embodiment, the solvent (b) is selected from the group consisting of water, propylene glycol, isopropanol and mixtures thereof

The composition preferably comprises, based on the total weight of the composition, 30 to 99.9 wt.-%, more preferably 40 to 99 wt.-% and most preferably 50 to 96 wt.-% like 50 to 90 wt.-% of solvent (b).

In particular, based on the total weight of the composition, 35 to 80 wt.-%, more preferably 35 to 70 wt.-% and most preferably 35 to 60 wt.-% of water and/or 0.5 to 30 wt.-%, more preferably 1 to 25 wt.-% and most preferably 10 to 20 wt.-% of propylene glycol and/or 5 to 15 wt.-%, more preferably 7 to 12 wt.-% and most preferably 9 to 10 wt.-% of isopropanol is used as solvent (b).

The composition also comprises at least one penetration enhancer (c). Penetration enhancers are pharmaceutically acceptable substances improving absorption of active substances via the skin. Extensive scientific studies have been carried out on this topic, essentially summarized in Massimiliano Nino's 2010 review (Massimiliano Nino, Gabriella Calabrò, Pietro Santoianni, Topical delivery of active principles: The field of dermatological research, Dermatology Online Journal, 16(1), 2010). The penetration enhancer (c) can be selected from skin penetration enhancers described in Osborne et al., Pharmaceutical Technology, 1997, 58 such as fatty alcohols (e.g. aliphatic alcohols, decanol, lauryl alcohol, linolenyl alcohol), fatty acid esters (e.g. butyl acetate, cetyl lactate, decyl N,N-dimethylamino acetate, diethylenglycol oleate), fatty acids (e.g. alkanoic acids, capric acid diacid etyloctadecanoic acid), fatty alcohol ethers (e.g. α-monoglyceryl ether, EO-2-oleyl ether, EO-5-oleyl ether, EO-10-oleyl ether), L-α-amino-acids, lecithin, phospholipids, saponin/phospholipids, sodium deoxycholate, sodium taurocholate, sodium tauroglycocholate, enzymes (e.g. acid phosphatase, calonase, orgelase, papain), amines and amides (e.g. acetamide derivatives, acyclic amides, N-adamantyl n-alkanamides, clofibric acid amides), complexing agents (e.g. β- and γ-cyclodextrin complexes, hydroxypropyl methylcellulose, liposomes, naphthalene diamide diimide), macrocyclics (e.g. macrocyclic lactones, ketones and anhydrides) classical surfactants (e.g. Brij 30-98, cetyl trimethyl ammonium bromide, Empicol ML26/F, HCO-60 surfactant), aliphatic thiols, alkyl N,N-dialkyl-substituted amino acetates, anise oil" eugenol, menthol, vitamin E, polar lipids, N-methyl pyrrolidone and related compounds (e.g. N-cyclohexyl-2-pyrrolidone, 1-butyl-3-dodecyl-2-pyrrolidone) ionic compounds (e.g. ascorbate, sodium carboxylate, sodium hyaluronate, sodium lauryl sulphate), dimethyl sulfoxide and related compounds, azones and related compounds (e.g. N-acyl-hexahydro-2-oxo-1H-azepines) and mixtures thereof.

Preferably, the penetration enhancer is selected from the group consisting of propylene glycol, phospholipids, lecithin, alcohols, DMSO (dimethylsulfoxide), DMAC (dimethylacetamide), DMFA (dimethylformamide),1-dodecyl azacycloheptan-2-one (Azone^{®}), diethylen glycol monoethyl ether (Transcutol^{®}CG), Macrogol 7 glycerol cocoate and mixtures thereof. More preferably, propylene glycol, phospholipids, lecithin, Macrogol 7 glycerol cocoate or mixtures thereof can be used as penetration enhancer (c).

Examples of suitable phospholipids can be selected from the group consisting of phosphatidylcholine, phosphatidylethanolamine, phosphatidylinosite, phosphatidylserine, lysophosphatidylcholine, phosphatidylglycerol, lysophosphatidylethanolamine, lysophosphatidylserine, lysophosphatidylinosite and mixtures thereof as well as salts and/or derivatives thereof. Derivatives can for example be formed by complete or partial hydrogenation of suitable phospholipids. Suitably, lecithin such as soya lecithin or egg lecithin like soya lecithin granules or soya lecithin solution in ethanol can be used as source of phospholipids. Alternatively or in addition to lecithin, synthetic phospholipids can be used.

The composition preferably comprises, based on the total weight of the composition, 3 to 40 wt.-%, more preferably 10 to 30 wt.-% and most preferably 15 to 25 wt.-% of penetration enhancer (c).

In particular, based on the total weight of the composition, 0.5 to 30 wt.-%, more preferably 1 to 25 wt.-% and most preferably 10 to 20 wt.-% of propylene glycol and/or 2 to 14 wt.-%, more preferably 3 to 10 wt.-% and most preferably 4 to 8 wt.-% of phospholipids is used as penetration enhancer (c).

In addition to components (a) to (c), the composition according to the invention can comprise at least one other excipient selected from the group consisting of preservatives, solubilizers, lipid-regulating excipients, buffer substances, anti-oxidants, gelling agents and mixtures thereof.

Examples of preservatives useful in the composition according to the present invention include, but are not restricted to pharmaceutically acceptable substances having antimicrobial effects, such as alcohols like ethanol, isopropanol, benzyl alcohol, chlorobutanol, bronopol etc., propylene glycol, parabens like methyl paraben, ethyl paraben etc., benzoic acid, sodium benzoate, sorbic acid, sodium sorbate, quaternary ammonium compounds like cetrimide, benzalkonium chloride, cetylpyridinium chloride, benzaethonium chloride, organic mercurials like thiomersal, phenylmercuric acetate etc., silver, sodium silver chloride, phenolics like cresol, chlorocresol, bisphenol etc. and mixtures thereof.

Examples of suitable solubilizers include triglycerides, glycolipids and phospholipids such as phosphatidylcholine, phosphatidylethanolamine, phosphatidylinosite, phosphatidylserine, lysophosphatidylcholine, phosphatidylglycerol, lysophosphatidylethanolamine, lysophosphatidylserine, lysophosphatidylinosite and mixtures thereof as well as salts and/or derivatives thereof including derivatives formed by complete or partial hydrogenation of suitable phospholipids. Preferably, lecithin such as soya lecithin granules or soya lecithin solution in ethanol can be used as source of phospholipids.

As used herein, the term "lipid-regulating excipients" refers to pharmaceutically acceptable excipients reducing the loss of moisture from the skin and/or preserving or restoring a sufficient layer of skin lipids. Examples of suitable lipid-regulating excipients include Macrogol 7 glycerol cocoate, ceramides, cholesterol and saturated fatty acids. It has been found that Macrogol 7 glycerol cocoate is a particularly preferred lipid-regulating excipient. Preferably, based on the total weight of the composition, 0 to 15 wt.-%, more preferably 6 to 12 wt.-% and most preferably 7 to 10 wt.-% of lipid-regulating excipient such as Macrogol 7 glycerol cocoate is used.

Examples of suitable buffer substances include phosphate buffers like sodium dihydrogen phosphate / disodium hydrogen phosphate, citric acid / sodium citrate and acetic acid and sodium acetate.

Examples of suitable antioxidants include uric acid, ascorbic acid, glutathione, melatonin, tocopherols, to-cotrienols, lecithin, citric acids as well as derivatives and mixtures thereof.

Optionally, a gelling agent like those conventionally used in pharmaceutical and cosmetic industry can be present in the pharmaceutical composition according to the invention. Examples of suitable gelling agents include carboxyvinyl polymers, hydroxyethyl cellulose, hydroxypropyl cellulose, hydroxypropyl methylcellulose, carboxymethyl cellulose, alginic acid-propylene glycol ester, polyacrylic acid polymers, carbomers and mixtures thereof.

In a preferred embodiment, the pharmaceutical composition according to the invention has a pH value ranging from 3 to 11, preferably more than 5.5 to 10.5 and even more preferably 6 to 10.5 like 6 to 8. Preferably, the pH is adjusted by using a pharmaceutically acceptable buffer system optionally in combination with a base like sodium hydroxide, an acid like hydrochloric acid or mixtures thereof.

It has surprisingly been found that the pharmaceutical composition according to the invention provides a particularly skin-compatible and safe form of application of tarenflurbil. Even though tarenflurbil is to be rated a comparatively well tolerated active ingredient, side effects still have to be expected with systemic administration. However, when administered in form of the pharmaceutical composition according to the invention tarenflurbil is transported to its desired site of action in the living cells of the dermis in a targeted manner and the systemic availability is kept as low as possible. Thus, although absorption of substances with higher molecular weight, such as tarenflurbil with a molecular weight of 244.26 g/mol, is generally made very difficult or prevented by the stratum corneum (horny skin), the very function and object of which is to act as a barrier against external influences and the absorption of foreign substances, the pharmaceutical composition according to the invention allows the tarenflurbil to be transported successfully through the stratum corneum into the living skin cell layers. At the same time the systemic availability of tarenflurbil is kept very low and, therefore, undesired side-effects are avoided by the pharmaceutical composition according to the invention.

Generally, a distinction is to be drawn between penetration and permeation of an active ingredient into or through the skin. Penetration is the entry of an active ingredient through the stratum corneum, the essential component of the skin barrier, into the epidermis (Dermatologie und Venerologie, Ed. Braun-Falco et al. 5th edition, 2005, p. 1431). Penetration of an active ingredient is desired in the treatment of skin diseases. Permeation is the transepidermal and transfollicular diffusion of active substances through the skin (Dermatologie und Venerologie, Ed. Braun-Falco et al. 5th edition, 2005, p. 1432). Permeation of an active ingredient is desired if absorption into the blood plasma and/or tissues situated under the skin (muscles, joints, ligaments, organs etc) via the skin is to be achieved.

Hence, the pharmaceutical composition according to the invention is characterized by a high penetration (transport of tarenflurbil through the stratum corneum into the living skin cell layers) and low permeation (systemic availability of tarenflurbil by absorption into the blood plasma and/or subcutaneous tissues via the skin) of tarenflurbil. In particular, the pharmaceutical composition according to the invention is characterized in that the blood plasma concentration of tarenflurbil (Cₘₐₓ) obtained after topical administration of the composition is less than 30%, preferably less than 20% and more preferably less than 15% of the tarenflurbil blood plasma concentration (Cₘₐₓ) obtained after administration of a systemic reference composition comprising the same amount of tarenflurbil. Preferably, the reference composition is an intravenous tarenflurbil injection solution.

Thus, the specific combination of excipients used in the pharmaceutical composition according to the invention results in that tarenflurbil is transported to its site of action into the living cell layers of the dermis without damaging the skin's barrier function and without providing a relatively high systemic availability of tarenflurbil. Moreover, it has been found that the tarenflurbil remains dissolved in the pharmaceutical composition according to the invention, even at low temperatures or after application of the composition onto the skin to allow penetration into the skin.

According to a preferred embodiment, the composition according to the invention comprises
- tarenflurbil or a pharmaceutically acceptable salt, solvate or ester thereof,
- water,
- propylene glycol,
- isopropanol,
- phospholipids like lecithin and
- optionally Macrogol 7 glycerol cocoate.

According to a particularly preferred embodiment, the composition according to the invention comprises
- tarenflurbil or a pharmaceutically acceptable salt, solvate or ester thereof,
- water,
- propylene glycol,
- isopropanol,
- phospholipids like lecithin,
- Macrogol 7 glycerol cocoate,
- sodium dihydrogen phosphate
- disodium hydrogen phosphate and
- sodium hydroxide.

It is particularly preferred that the composition according to invention comprises, based on the total weight of the composition,
- 0.10 to 5.00 wt.-% of tarenflurbil or a pharmaceutically acceptable salt, solvate or ester thereof,
- 36.00 to 60.00 wt.-% of water,
- 1.00 to 25.00 wt.-% of propylene glycol,
- 5.00 to 15 wt.-% of isopropanol,
- 1.00 to 14.00 wt.-% of phospholipids like lecithin and
- 0 to 15.00 wt.-% of Macrogol 7 glycerol cocoate.

It is also particularly preferred that the composition according to invention comprises, based on the total weight of the composition,
- 0.10 to 5.00 wt.-% of tarenflurbil or a pharmaceutically acceptable salt, solvate or ester thereof,
- 36.00 to 60.00 wt.-% of water,
- 1.00 to 25.00 wt.-% of propylene glycol,
- 5.00 to 15 wt.-% of isopropanol,
- 2.00 to 14.00 wt.-% of lecithins like soya lecithin,
- 0 to 15.00 wt.-% of Macrogol 7 glycerol cocoate,
- 0.25 wt.-% of sodium dihydrogen phosphate,
- 0.57 wt.-% of disodium hydrogen phosphate and
- 0 to 4.00 wt.-% of sodium hydroxide.

The pharmaceutical composition according to the invention can be prepared by any method known in the art. In one embodiment, the ingredients of the composition are mixed, preferably at room temperature, and then a base such as sodium hydroxide is added to adjust the pH of the resulting mixture to 4.5 to 10.5, preferably 6 to 10.5, more preferably about 7.5.

The pharmaceutical composition according to the invention is preferably for use in treatment of skin diseases. More preferably, the pharmaceutical composition according to the invention is for use in the treatment of skin diseases according to the first aspect of the invention as well as psoriasis and seborrheic dermatitis.

Hence, the pharmaceutical composition according to the second aspect of the invention is particularly for use in the treatment of a skin disease selected from the group consisting of erythematous diseases, papular and lichenoid diseases (including lichen rubber like lichen rubber planus, lichenoid exanthema, graft-versus-host disease of the skin), pustular diseases, UV erythema, atopic dermatitis, urticaria (including acute urticaria, chronic urticaria, autoimmune urticaria, physical urticaria like pressure urticaria, heat urticaria, cold urticaria and light urticaria, contact urticaria, adrenergic urticaria and aquagenic urticaria), angioedema, contact dermatitis (including irritant dermatitis like acute toxic contact eczema and chronic toxic contact eczema, and allergic contact dermatitis like acute allergic contact dermatitis and chronic allergic contact eczema), dyshidrosis, pruritus, prurigo, acne, rosacea, psoriasis and seborrheic dermatitis. Preferably, atopic dermatitis, psoriasis, UV erythema, pruritus, allergic skin diseases, such as allergic contact dermatitis and allergic urticaria, and acne can be treated with the pharmaceutical composition according to the invention.

The invention is now illustrated in more detail by the following examples and figures which are not intended to limit the scope of the claims.
Figure 1 shows the leukotriene synthesis (group determination of LT-C₄/E₄/D₄) in the case of incubation with various tarenflurbil dosages according to Example 1.
Figure 2a shows the chromametry results obtained in Example 4 for a 3% tarenflurbil gel.
Figure 2b shows the skin blood flow obtained in Example 4 during treatment with a 3% tarenflurbil gel.
Figure 3a shows the chromametry results obtained in Example 4 for a 5% flurbiprofen gel.
Figure 3b shows the skin blood flow obtained in Example 4 during treatment with a 5% flurbiprofen gel.
Figure 4 shows the field scores of the subjects TK, FR and VK, during treatment of a contact eczema according to Example 5, with LK = Chamber control, VH = Vehicle control, MF = Mometasone furoate, and TF = Tarenflurbil.
Figure 5 shows the ranking scores of the subjects TK, FR and VK during treatment of a contact eczema according to Example 5, with LK = Chamber control, VH = Vehicle control, MF = Mometasone furoate, and TF = Tarenflurbil.

### Example 1

In an *in vitro* whole blood culture test system with human whole blood from 3 donors in each case, pre-incubation was carried out for 2 hours with 0.39, 1.56, 6.25, 25 and 100 µg tarenflurbil/ml whole blood before activation with zymosan (agent for producing sterile inflammations) took place. In total the following 37 parameters were measured which allow a broad overview of the reaction of the leucocytes under inflammatory conditions and thus also of the effects of tarenflurbil in such an environment:
1. Amphiregulin
2. Brain-derived neurotrophic factor (BDNF)
3. Interleukin-1 alpha (IL-1α)
4. IL-1β
5. IL-1 receptor antagonist (IL-1Ra)
6. IL-2
7. IL-3
8. IL-4
9. IL-5
10. IL-6
11. IL-7
12. IL-8
13. IL-10
14. IL-12, p40 subunit (IL-12p40)
15. IL-12p70
16. IL-15
17. IL-17
18. IL-18
19. IL-23
20. Epiregulin
21. Eotaxin (Eot.)
22. Factor VII (F. VII)
23. Granulocyte-monocytes colony stimulating factor (GM-CSF)
24. Interferon gamma (IFNγ)
25. Intercellular adhesion molecule-1 (ICAM-1)
26. Monocyte chemoattractand protein-1 (MCP-1)
27. Migration inhibitory protein-1 alpha (MIP-1α)
28. MIP-1ß
29. Matrix-metalloproteinase-2 (MMP-2)
30. MMP-3
31. MMP-9
32. Oncostatin M (OSM)
33. Stem-cell factor (SCF)
34. Tissue growth factor alpha (TGFα)
35. Tumor necrosis factor alpha (TNFα)
36. TNFß
37. Vascular endothelial growth factor (VEGF)

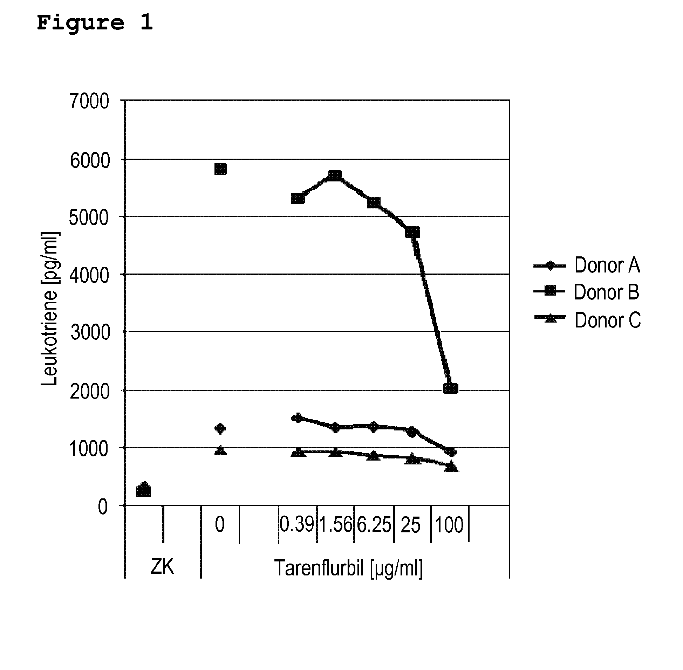

Among the extensive results, it has been found that taren-furbil
- effects a dose-dependent reduction in PGE2 (prostaglandin E2) formation,
- effects a dose-dependent reduction in leukotriene synthesis (LT-C4/E4/D4) in allergy sufferers (see Figure 1),
- effects a dose-dependent reduction in TGFß (transforming growth factor ß) release, and
- effects a dose-dependent reduction in BDNF (brain-derived neurotrophic factor) release.

Prostaglandin E2 plays an important role in the case of inflammatory skin diseases and is elevated (Kanda et al., Prostaglandin E2 Enhances Neurotrophin-4 Production via EP3 Receptor in Human Keratinocytes, The Journal of Pharmacology and Experimental Therapeutics, 315(2), 2005, 796-804). Prostaglandin E2 is in addition also jointly responsible for the occurrence of itching (pruritus) (Philippe Furger, Innere quick: der Fakten-Turbo, Georg Thieme Verlag, 2003, p. 480). It has surprisingly been found that tarenflurbil - although it does not have any cyclooxygenase inhibitory activity - results in the obtained reduction in PGE2 formation. Thus, it has unexpectedly been shown that even without cyclooxygenase inhibitory activity tarenflurbil is in principle useful for the treatment of inflammatory skin diseases via reduction in PGE2 formation.

Leukotrienes, products of arachidonic acid metabolism, play a central role in the development of inflammatory processes. The catalytic effect of 5-lipoxygenase leads to the production of the inflammation mediators cysteinyl leukotrienes C4, D4 and E4. There is still little data on the treatment of atopic dermatitis with leukotriene antagonists, however, the existing findings already indicate a moderate but significant improvement in the disease compared with placebo. In addition, the systemic inhibition of the arachidonic acid metabolism lead to a decrease in the total epidermal lipids and the pro-inflammatory lipid fractions which are considered responsible for the development of inflammatory acne lesions (Zouboulis CC, Leukotrienantagonisten bei atopischen Erkrankungen und Akne, Akt Dermatol 2003, 419-425).

It has surprisingly been found that besides PGE2 inhibitory activity tarenflurbil also exhibits leukotriene inhibitory activity. Thus, besides PGE2 tarenflurbil is also capable of inhibiting a second major class of mediators of inflammation.

Figure 1 shows the obtained Leukotriene synthesis during incubation with the various tarenflurbil dosages. A group determination of LT-C₄/E₄/D₄ was carried out. The specific characteristic of Donor B is that this is the only allergy sufferer of the three individuals. Thus, the dose-dependent reduction in leukotriene synthesis obtained for Donor B indicates a very selective development of the effect of tarenflurbil.

The BDNF concentration is greatly increased in patients with atopic eczema (Hoffjan S. et al., Variation in the BDNF and NGFß genes in German atopic dermatitis patients, Mol Cell Probes. 2009, 23(1), 35-38). Therefore, the obtained dose-dependent BDNF reduction during administration of tarenflurbil is expected to have a positive effect on the symptoms of atopic dermatitis.

### Example 2

The clinical-pharmacological studies of the following Examples 2 to 4 were carried out on healthy adults as model for non-immunological inflammation.

In a preliminary test on the skin of the back involving a group of four test subjects, the "minimum erythema dose" following irradiation with a standardized daylight irradiation apparatus (OSRAM VITALUX) was established. In the main test 8 areas of the skin of the back of each subject were irradiated with twice the "minimum erythema dose" and then treated with tarenflurbil spray with 0.1, 0.25, 1 and 4% tarenflurbil (w/w) as well as the associated placebo formulations and the comparison preparations (triamcinolone acetonide 0.2% (w/w); mometasone furoate solution 0.1% (w/w)). The used test preparations are summarized in the following table:

| **Name** | **Random code** | **Preparation** |
|---|---|---|
| Test 1 | E | Tarenflurbil spray 4% (w/w), (5% lecithin) |
| Test 2 | I | Tarenflurbil spray 1% (w/w), (5% lecithin) |
| Test 3 | B | Tarenflurbil spray 0.25% (w/w), (5% lecithin) |
| Test 4 | A | Tarenflurbil spray 0.1% (w/w), (5% lecithin) |
| Test 5 | D | Tarenflurbil spray 1% (w/w), (8% lecithin) |
| Placebo 1 | G | Tarenflurbil spray PLACEBO, (5% lecithin) |
| Placebo 2 | J | Tarenflurbil spray PLACEBO, (8% lecithin) |
| Comparison solution 1 | F | Triamgalen^{®} solution 0.2% (w/w), triamcinolone acetonide |
| Comparison solution 2 | C | Ecural^{®} solution 0.1% (w/w), mome-tasone furoate |

After times 0, 1, 2, 3, 5, 7, 9, 24, 32, and 48 hours after the application of the test preparations, the degree of erythema was established by means of a visual score and a spectroscopic colour measurement. Tarenflurbil showed a dose-dependent reduction in the UV erythema (sunburn). The effect of the test preparation with the highest tarenflurbil concentration was much more marked than with the comparison solutions triamcinolone acetonide 0.2% (w/w) or mometasone furoate 0.1% (w/w).

### Example 3

In a further test with 6 volunteers, a 10% (w/w) tarenflurbil spray relative to its placebo and a 3% (w/w) tarenflurbil gel were administered following irradiation with a single minimum erythema dose and compared with each other. The 10% (w/w) tarenflurbil spray and the 3% (w/w) tarenflurbil gel showed a clearly marked anti-erythematous effect.

### Example 4

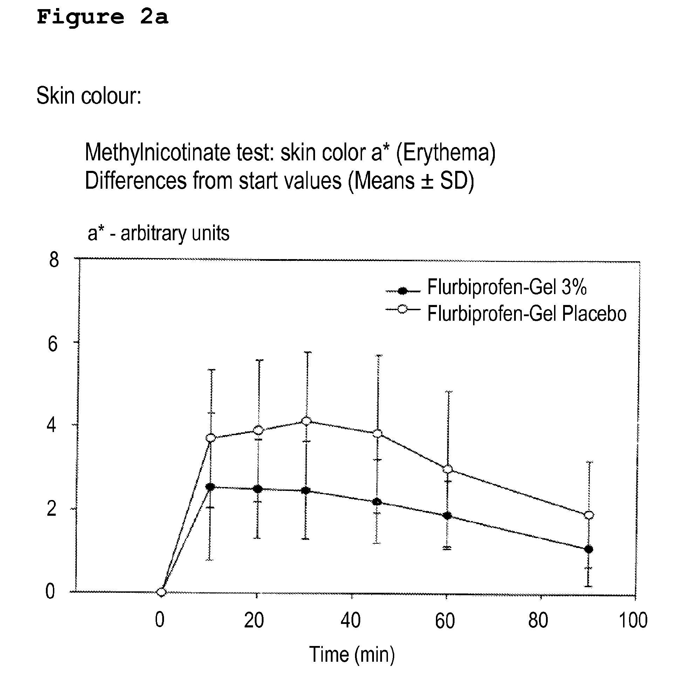

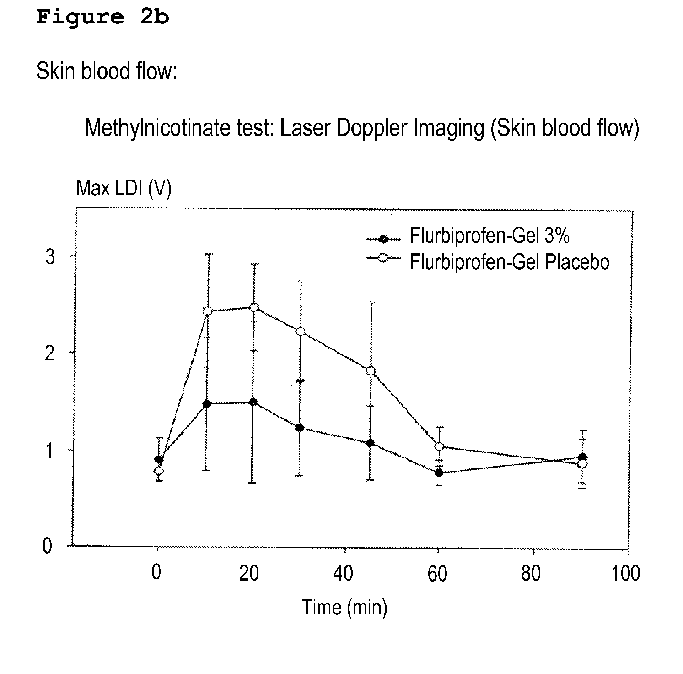

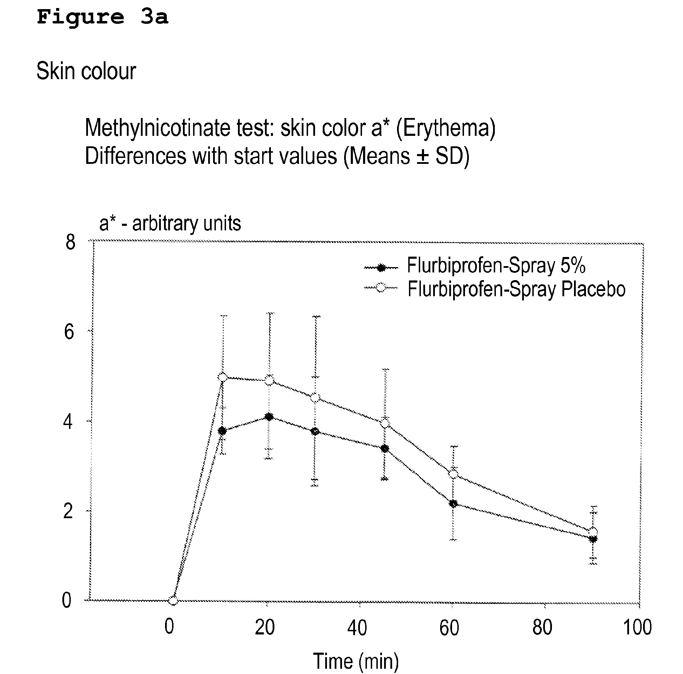

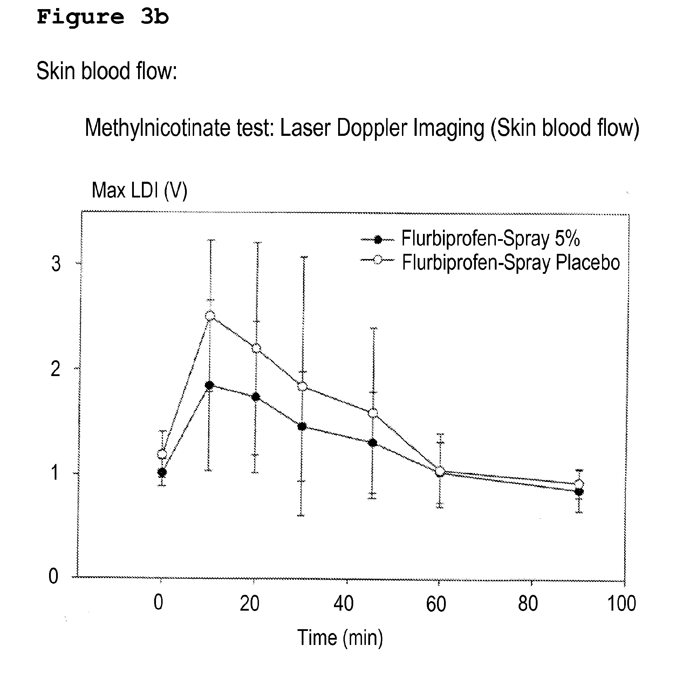

A methyl nicotinate test was carried out in a test with 5 subjects. The test preparations (3% (w/w) tarenflurbil gel, 5% (w/w) tarenflurbil solution/spray) as well as their placebo formulations were applied twice daily over 5 days to marked surfaces on the inside of the forearm of the subjects. 30 minutes after the last application on the 5th day, the methyl nicotinate test was carried out by applying methyl nicotinate (10 mM in water) to the marked surfaces which usually causes erythema of the skin. The anti-erythematous effect of the test preparations was ascertained via a spectroscopic skin colour measurement (chromametry) and a laser Doppler image analysis (skin blood flow) up to 90 minutes after the provocation. Figures 2a and 3a show the obtained chromametry results of 3% (w/w) tarenflurbil gel and 5% (w/w) tarenflurbil solution/spray, respectively. Figures 2b and 3b show the obtained skin blood flow results of 3% (w/w) tarenflurbil gel and 5% (w/w) tarenflurbil solution/spray, respectively. As can be seen from these Figures, both 3% (w/w) tarenflurbil gel and 5% (w/w) tarenflurbil solution/spray demonstrated a clearly marked anti-edematous effect in the spectroscopic skin colour test and the laser Doppler image analysis.

### Example 5

After efficacy of tarenflurbil in the case of non-immunological inflammatory skin reactions was confirmed in the preliminary tests on healthy subjects, in the next step it was tested whether tarenflurbil can also be used beneficially on patients with immunological-inflammatory skin diseases.

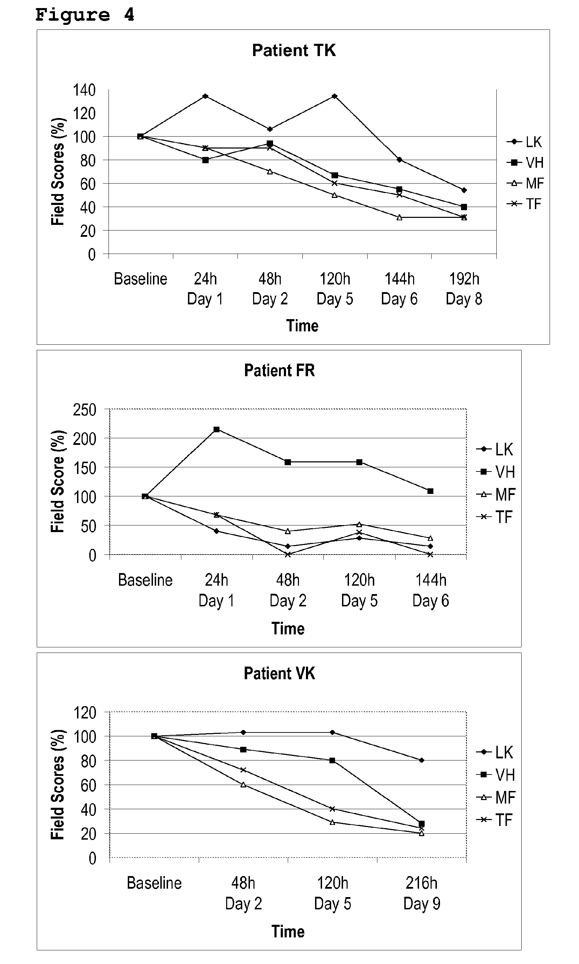

For this, patients with an allergic reaction to nickel were treated. A nickel allergy was evoked in 3 patients on the upper back in a patch test. In each case, contact eczema was triggered in 4 areas of the back skin of a patient by applying a nickel-containing (5% nickel(II) sulphate hexahydrate in white vaseline). One area position was treated with tarenflurbil solution according to Example 9 (containing 2 wt.-% of tarenflurbil) described below. A second position was treated with 0.05% (w/w) mometasone furoate solution as positive control, while a third was treated only with the placebo base of the tarenflurbil solution as negative control. The fourth test area remained untreated for control purposes. A treatment of the selected areas with the test preparations was carried out once daily over a period of 9 days. For the evaluation a "field score" and a "ranking score" were recorded and photo documentation was prepared. The "field score" first assessed individually on a five-point scale the symptoms erythema, oedema formation, pustule and blister formation, the surface area and the degree of dehydration of the allergic skin reaction; 0 meant "not present", and e.g. 4 "very serious". After completion of the thus-prepared individual assessments of the individual symptoms, these were totalled and divided by the maximum possible value, and represented as a relative percentage value, which then ultimately produced the final "field score" The obtained results are shown in Figure 4.

The starting values (baseline) were as follows:

| Patient TK | | Patient FR | | Patient VK | |
|---|---|---|---|---|---|
| LK | 37.5 | LK | 18.75 | LK | 43.75 |
| VH | 18.75 | VH | 37.5 | VH | 56.25 |
| MF | 50 | MF | 37.5 | MF | 50 |
| TF | 50 | TF | 37.5 | TF | 62.5 |

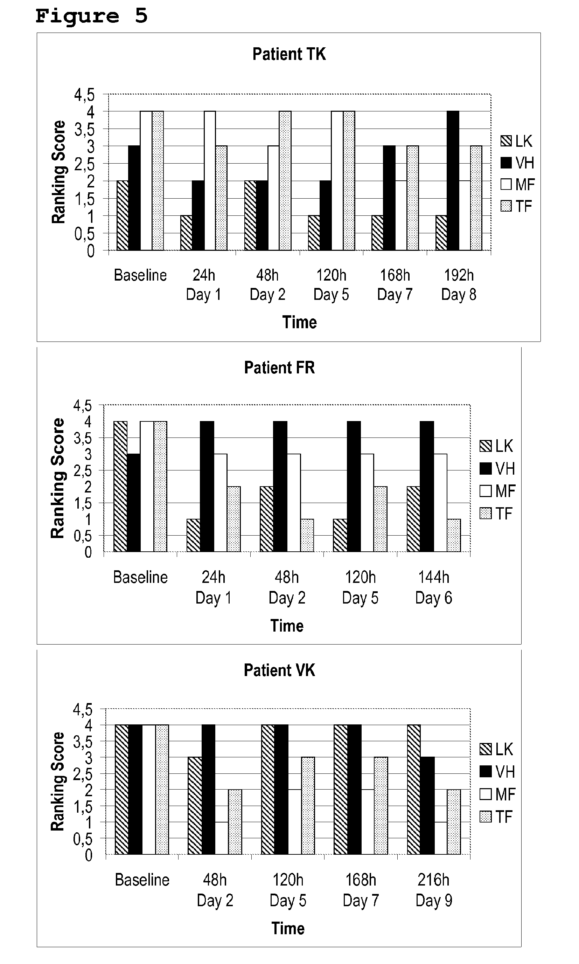

In order to compare the four different treatment fields with each other at the various test times, the "ranking score" was recorded. The procedure was as follows: For each test time, each of the four test areas was allocated a test figure of 1 to 4, with 1 standing for the most weakly marked lesion and 4 the most strongly marked lesion. The obtained ranking scores are shown in Figure 5. In the result, the effectiveness of the tested tarenflurbil solution proved at least equal to that of the corticoid test formulation and was clearly superior to the placebo. The effectiveness was most clearly ascertainable after 3 to 5 days of treatment.

### Example 6

2 patients with atopic eczema were treated in a second clinical therapy trial. Patient A, who had severe atopic dermatitis, was treated semilaterally for a period of 11 days twice daily with tarenflurbil solution according to Example 9 (containing 2 wt.-% of tarenflurbil), and on the contralateral side with 0.1% (w/w) triamcinolone cream (TRIAMGALEN® creme), a corticoid. The PGA score (physician's global rating) for the classification of the clinical activity was recorded with a seven-step scale, with 0 standing for "no symptoms" and 6 for "very severe symptoms". The PGA score improved by 2 for both medicaments (tarenflurbil from 5 to 3 and the corticoid from 4 to 2). Thus, the tarenflurbil formulation proved to be comparable with the Class II corticoid. No side effects were recorded when applying the tarenflurbil formulation.

Patient B, who had moderately severe atopic dermatitis, was treated for a period of 14 days on one side on the inside of the left elbow twice daily with tarenflurbil solution according to Example 9 (containing 2 wt.-% of tarenflurbil). The PGA score improved from 4 to 1. The tarenflurbil formulation proved to be very effective. No side effects were recorded when applying the tarenflurbil formulation.

### Example 7

In a further clinical therapy trial, 9 patients with atopic dermatitis were treated in the half-side test twice daily with tarenflurbil solution according to Example 9 (containing 2 wt.-% of tarenflurbil) and twice daily 0.1% (w/w) triamcinolone lotion (TRIAMGALEN^{®} lotion). The lesions occurred on different skin areas (e.g. knee, wrist, elbow etc.) and in each case the symmetrically opposite areas were treated with the named preparations. In this case, too, the PGA score was recorded. In five out of nine patients the efficacy of the 2% (w/w) tarenflurbil formulation proved comparable with that of the Class II corticoid as can be seen from the following table.

| | Week 0 PGA | Week 2 PGA | Week 4 PGA | Week 6 PGA |
|---|---|---|---|---|
| Patient 1, male, 28y | 5^{a} | 3^{a} | | |
| | 4^{b} | 2^{b} | | |
| Patient 3, male, 25y | 3^{a} | 1^{a} | | |
| | 4^{b} | 0^{b} | | |
| Patient 4, female, 28y | 4^{a} | 0^{a} | | |
| | 3^{b} | 0^{b} | | |
| Patient 8, female, 7y | 2^{a} | 1^{a} | | |
| | 2^{b} | 2^{b} | | |
| Patient 9, male, 38y | 4^{a} | 2^{a} | | |
| | 4^{b} | 1^{b} | | |
| Patient 2, male, 64y | 3^{a} | 4^{a} | 0^{a} | |
| | 4^{b} | 1^{b} | 0^{b} | |
| Patient 6, female, 21y | 4^{a} | 5^{a} | 1 | |
| | 2^{b} | 1^{b} | 1^{b} | |
| Patient 5, male, 30y | 5^{a} | 4^{a} | | 4^{a} |
| | 5^{b} | 1^{b} | | 0^{b} |
| Patient 7, male, 35y | 3^{a} | 2^{a} | | 1^{a} |
| | 4^{b} | 0^{b} | | 0^{b} |

| | | | | |
|---|---|---|---|---|
| ^{a}: Treatment with 2% (w/w) tarenflurbil spray ^{b}: Treatment with 0.1% (w/w) triamcinolone lotion | | | | |

### Example 8

A patient with psoriasis capitis in the region of the hair line, with overlaid seborrhoic eczema, complained of severe itching in this area over an extended period. Following application of a tarenflurbil solution according to Example 9 (containing 4 wt.-% of tarenflurbil) the itching clearly abated and reappeared after the therapy was discontinued. In a subsequent half-side test (one half of the diseased area was treated, the other not) it was established that the itching abated only on the treated surface.

Another patient, with mild psoriasis in the facial region, successfully applied a tarenflurbil solution according to Example 9 (containing 4 wt.-% of tarenflurbil) over 1.5 years and completely dispensed with the topical corticoid formulations used constantly until then.

No drug-induced side effects occurred in any of the above-mentioned clinical-pharmacological trials with tarenflurbil preparations and the tolerability of these tarenflurbil medicaments was rated excellent by the testers.

### Example 9

In the following Examples 9 to 15, the first mentioned ingredients up to and including disodium hydrogen phosphate dodecahydrate were mixed together and stirred at room temperature for 140 minutes. Then the pH value was set at pH 7.5 with the NaOH solution followed by addition of the remaining quantity of water. The pH of the resulting composition varied within the range of pH 7.5 +/- 3. In each example, four different compositions having varying tarenflurbil concentrations were prepared.

| **Ingredient** | **Composition in wt.-%** |
|---|---|
| Tarenflurbil | 0.25, 1, 2, 4 |
| propylene glycol | 15.00 |
| isopropyl alcohol | 9.35 |
| soya lecithin granules | 5.00 |
| Macrogol 7 glycerol cocoate | 8.60 |
| purified water | 56.00 |
| sodium dihydrogen phosphate dehydrate | 0.25 |
| disodium hydrogen phosphate dodecahydrate | 0.57 |
| sodium hydroxide solution in water (20% w/w) | 0.0-2.40 |
| purified water | ad 100.00 |

### Example 10

| **Ingredient** | **Composition in wt.-%** |
|---|---|
| Tarenflurbil | 0.25, 1, 2, 4 |
| propylene glycol | 15.00 |
| isopropyl alcohol | 15.00 |
| soya lecithin granules | 2.00 |
| purified water | 56.00 |
| sodium dihydrogen phosphate dehydrate | 0.25 |
| disodium hydrogen phosphate dodecahydrate | 0.57 |
| sodium hydroxide solution in water (20% w/w) | 0.0-2.40 |
| purified water | ad 100.00 |

### Example 11

| **Ingredient** | **Composition in wt.-%** |
|---|---|
| Tarenflurbil | 0.25, 1, 2, 4 |
| propylene glycol | 15.00 |
| isopropyl alcohol | 5.00 |
| soya lecithin granules | 14.00 |
| Macrogol 7 glycerol cocoate | 8.60 |
| purified water | 56.00 |
| sodium dihydrogen phosphate dehydrate | 0.25 |
| disodium hydrogen phosphate dodecahydrate | 0.57 |
| sodium hydroxide solution in water (20% w/w) | 0.0-2.40 |
| purified water | ad 100.00 |

### Example 12

| **Ingredient** | **Composition in wt.-%** |
|---|---|
| Tarenflurbil | 0.25, 1, 2, 4 |
| propylene glycol | 10.00 |
| isopropyl alcohol | 5.00 |
| soya lecithin granules | 7.00 |
| Macrogol 7 glycerol cocoate | 15.00 |
| purified water | 46.00 |
| sodium dihydrogen phosphate dehydrate | 0.25 |
| disodium hydrogen phosphate dodecahydrate | 0.57 |
| sodium hydroxide solution in water (20% w/w) | 0.0-2.40 |
| purified water | ad 100.00 |

### Example 13

| **Ingredient** | **Composition in wt.-%** |
|---|---|
| Tarenflurbil | 0.25, 1, 2, 4 |
| propylene glycol | 10.00 |
| isopropyl alcohol | 5.00 |
| soya lecithin solution (75% w/w in ethanol) | 4.00 |
| Macrogol 7 glycerol cocoate | 6.60 |
| purified water | 56.00 |
| sodium dihydrogen phosphate dihydrate | 0.25 |
| disodium hydrogen phosphate dodecahydrate | 0.57 |
| sodium hydroxide solution in water (20% w/w) | 0.0-2.40 |
| purified water | ad 100.00 |

### Example 14

| **Ingredient** | **Composition in wt.-%** |
|---|---|
| Tarenflurbil | 0.25, 1, 2, 4 |
| propylene glycol | 25.00 |
| Isopropyl alcohol | 5.00 |
| Soya lecithin solution (75% w/w in ethanol) | 4.00 |
| Macrogol 7 glycerol cocoate | 6.60 |
| purified water | 36.00 |
| sodium dihydrogen phosphate dihydrate | 0.25 |
| disodium hydrogen phosphate dodecahydrate | 0.57 |
| sodium hydroxide solution in water (20% w/w) | 0.0-2.40 |
| purified water | ad 100.00 |

### Example 15

| **Ingredient** | **Composition in wt.-%** |
|---|---|
| Tarenflurbil | 0.25, 1, 2, 4 |
| propylene glycol | 1.00 |
| isopropyl alcohol | 5.00 |
| soya lecithin solution (75% w/w in ethanol) | 4.00 |
| Macrogol 7 glycerol cocoate | 6.60 |
| purified water | 56.00 |
| sodium dihydrogen phosphate dihydrate | 0.25 |
| disodium hydrogen phosphate dodecahydrate | 0.57 |
| sodium hydroxide solution in water (20% w/w) | 0.0-2.40 |
| purified water | ad 100.00 |

## Claims

1. Tarenflurbil or a pharmaceutically acceptable salt, solvate or ester thereof for use in the treatment of inflammation and/or itching associated with a skin disease.

2. Tarenflurbil or a pharmaceutically acceptable salt, solvate or ester thereof according to claim 1, wherein the skin disease is a non-hyperproliferative skin disease.

3. Tarenflurbil or a pharmaceutically acceptable salt, solvate or ester thereof according to claim 1 or 2, wherein the skin disease is treated by inhibiting at least one inflammation mediator selected from the group consisting of prostaglandins, leukotrienes, BDNF and TGFβ.

4. Tarenflurbil or a pharmaceutically acceptable salt, solvate or ester thereof according to any one of claims 1 to 3, wherein the skin disease to be treated is associated with IgE elevation and/or increased Th2 cytokine expression.

5. Tarenflurbil or a pharmaceutically acceptable salt, solvate or ester thereof according to claims 1, 3 or 4, wherein the skin disease to be treated is selected from the group consisting of erythematous diseases, papular and lichenoid diseases, pustular diseases, psoriasis, UV erythema, atopic dermatitis, urticaria, angioedema, contact dermatitis, dyshidrosis, pruritus, prurigo, acne and rosacea.

6. Tarenflurbil or a pharmaceutically acceptable salt, solvate or ester thereof according to claim 5, wherein the skin disease to be treated is selected from the group consisting of atopic dermatitis, psoriasis, UV erythema, pruritus, allergic skin diseases and acne.

7. Tarenflurbil or a pharmaceutically acceptable salt, solvate or ester thereof according to claim 6, wherein the skin disease to be treated is atopic dermatitis.

8. Tarenflurbil or a pharmaceutically acceptable salt, solvate or ester thereof according to any one of claims 1 to 7, wherein the tarenflurbil or a pharmaceutically acceptable salt, solvate or ester thereof is in a form for topical administration.

9. Tarenflurbil or a pharmaceutically acceptable salt, solvate or ester thereof according to claim 8, wherein the tarenflurbil or a pharmaceutically acceptable salt, solvate or ester thereof is in a form of a topical pharmaceutical composition selected from the group consisting of liquid or semi-solid formulations and plasters.

10. Tarenflurbil or a pharmaceutically acceptable salt, solvate or ester thereof according to claim 9, wherein the topical pharmaceutical composition comprises tarenflurbil or a pharmaceutically acceptable salt, solvate or ester thereof and at least one penetration enhancer.

11. Tarenflurbil or a pharmaceutically acceptable salt, solvate or ester thereof according to claim 9 or 10, wherein the topical pharmaceutical composition is in a form of a solution.

12. Tarenflurbil or a pharmaceutically acceptable salt, solvate or ester thereof according to claim 11, wherein the topical pharmaceutical composition comprises tarenflurbil or a pharmaceutically acceptable salt, solvate or ester thereof, a solvent, a penetration enhancer and optionally further excipients.

13. Tarenflurbil or a pharmaceutically acceptable salt, solvate or ester thereof according to claim 12, wherein the topical pharmaceutical composition comprises tarenflurbil or a pharmaceutically acceptable salt, solvate or ester thereof, propylene glycol, isopropyl alcohol, phospholipids, optionally Macrogol 7 glycerol cocoate and optionally further excipients.

14. Tarenflurbil or a pharmaceutically acceptable salt, solvate or ester thereof according to any one of claims 1 to 6, wherein the tarenflurbil or a pharmaceutically acceptable salt, solvate or ester thereof is in a form for systemic administration.

15. Pharmaceutical composition of tarenflurbil comprising
(a) tarenflurbil or a pharmaceutically acceptable salt, solvate or ester thereof,
(b) at least one solvent,
(c) at least one penetration enhancer, and
(d) optionally further excipients.
